Europäisches Patentamt

European Patent Office

Office européen des brevets

(19)

(11) Veröffentlichungsnummer : **0 061 437**
**B1**

(12)                    EUROPÄISCHE PATENTSCHRIFT

(45) Veröffentlichungstag der Patentschrift :
18.06.86

(51) Int. Cl.⁴ : **G 01 N 21/55**

(21) Anmeldenummer : **82850043.9**

(22) Anmeldetag : **08.03.82**

(54) Infrarotanalysator, insbesondere für Lebensmittel wie Mehl.

(30) Priorität : **16.03.81 SE 8101655**

(43) Veröffentlichungstag der Anmeldung :
**29.09.82 Patentblatt 82/39**

(45) Bekanntmachung des Hinweises auf die Patenterteilung : **18.06.86 Patentblatt 86/25**

(84) Benannte Vertragsstaaten :
**CH DE FR GB IT LI**

(56) Entgegenhaltungen :
**US-A- 3 224 324**
**US-A- 3 321 636**
**US-A- 3 328 587**
**US-A- 3 817 628**
**US-A- 4 040 747**

(73) Patentinhaber : **Perten, Peter**
**Am Golfplatz 61**
**D-2070 Ahrensburg (DE)**

(72) Erfinder : **Perten, Peter**
**Am Golfplatz 61**
**D-2070 Ahrensburg (DE)**

(74) Vertreter : **Kierkegaard, Lars-Olov et al**
**H. ALBIHNS PATENTBYRA AB Box 7664**
**S-103 94 Stockholm (SE)**

**0 061 437**

**Beschreibung**

Die Erfindung betrifft einen Infrarotanalysator gemäss dem Oberbegriff des Hauptanspruchs.

Derartige Analysatoren sind schon seit langem für Materialanalyse gebräuchlich, besonders um bei Lebensmitteln das Vorkommen oder die relative (prozentuale) Menge gewisser Stoffe wie Wasser, Protein und Fett festzustellen. Eine Probe wird mit infrarotem Licht bestrahlt, meistens mit Licht verschiedener Wellenlänge der Reihe nach, und das von der Probe reflektierte Licht wird mit dem von einer Referenz (Bezugsmittel) reflektiertem Licht verglichen. Betreffs Genauigkeit müssen bei der Bestrahlung und Messung des reflektierten Lichts hohe Ansprüche erfüllt werden. Unter anderem darf der Strahlengang zwischen der Probe selbst und der lichtelektrischen Einrichtung — nachstehend Fotozelleneinrichtung genannt — nicht von Stoffen gestört werden, die das infrarote Licht beeinflussen, und wenn mehrere Fenster aus Glas o. ä. im genannten Teil des Strahlengangs vorgesehen werden, müssen sie aufeinander abgestimmt sein.

Die Probe besteht oft nicht aus einem festen steifen Körper oder festen groben Körnern, sondern beispielsweise aus Mehl oder anderem feinen Pulver, klebrigen Körnern, Algen oder einer schmierigen Masse wie Hackfleisch oder Wurstmasse. Es ist üblich, solche Proben in einem offenen Napf, Schale oder ähnlichem Behälter unterzubringen und dann zusammenzudrücken, bevor sie in den Analysator gelegt werden. Um zu verhindern, dass die zu bestrahlende Oberfläche der Probe sich während der Handhabung verformt, deckt man oft die Probe mit einer Glasscheibe, die auch zum Zusammendrücken der Probe benutzt werden kann. Solche schalenförmige Probebehälter lassen sich aber nur schwer füllen, entleeren und reinigen.

Ein Infrarotanalysator gemäss dem Oberbegriff des Anspruchs 1 wird in der US-A-4 040 747 gezeigt. Der dort in Fig. 2 gezeigte Probenbehälter hat eine die Probe deckende Glasscheibe und eine darunter befindliche, federnde Platte zum federnden Zusammendrücken der Probe. Dieser Probenbehälter ist ganz kompliziert und macht auch die Handhabung kompliziert und zeitraubend, da er zum Füllen, Entleeren und Reinigen zerschraubt und wieder zusammengeschraubt werden muss.

Um die Handhabung einfacher zu machen, benutzt man manchmal einfache offene Schalen, in denen die Probe von oben her bestrahlt werden muss, wenigstens wenn es sich um pulvrige, breiförmige, klebrigkörnige oder flüssige Stoffe handelt. Analysatoren dieser Art sind u. a. aus der US-A-3 776 642 und der US-A-4 236 076 bekannt. Nachteilig ist jedoch, dass die Bestrahlung von oben her geschehen muss.

Aufgabe der Erfindung ist, die genannten Nachteile zu eliminieren und einen verbesserten Infrarotanalysator zu schaffen, wo der Probenbehälter so ausgeführt ist, dass er sich leicht füllen, entleeren und reinigen lässt. Ausserdem soll ein erwünschtes Zusammendrücken des Probenmaterials leicht erreicht werden können. Es soll auch möglich sein, die Probe nicht notwendigerweise von oben her zu bestrahlen.

Gemäss der Erfindung wird diese Aufgabe dadurch gelöst, dass der Analysator die kennzeichnende Merkmale des Hauptanspruchs aufweist.

Die erfindungsgemässen Massnahmen bewirken, mit dem einfachen Behälter, eine wesentliche Vereinfachung der Handhabung des Analysators, und sie ermöglichen auch bessere Analysenergebnisse dadurch, dass das Lichtbündel der Lichtquelle auf das Fenster des Probebehälters direkt treffen kann. Es ist zudem möglich, einen erfindungsgemässen Analysator für nicht vertikale Bestrahlungsrichtungen zu benutzen.

Nachstehend werden erfindungsgemässe Ausführungsbeispiele anhand der anliegenden Zeichnung beschrieben. Fig. 1 der Zeichnung zeigt einen Analysator schematisch. Fig. 2 ist ein Längsschnitt durch einen Behälter für eine zu untersuchende Materialprobe. Fig. 3 veranschaulicht ein Beispiel eines zur Aufnahme der Probe bestimmten Teils eines Analysators. Fig. 4 zeigt einen Probenverdichter. Fig. 5 stellt eine Abart des Probenbehälters dar.

Es ist zu beachten, dass nachstehend einfachheitshalber das Wort « Mehl » (aus Getreide) für alle Arten Stoffe benutzt wird, die als zu untersuchende Probe infrage kommen, wenn nicht die gesamte Probe starr und fest ist und nicht aus groben festen Körnern besteht. Ferner wird der Ausdruck « Glas » für alle Feststoffe benutzt, die für infrarotes Licht durchlässig sind. Die üblichen Abkürzungen IR und UV beziehen sich auf infrarotes bzw. ultraviolettes Licht.

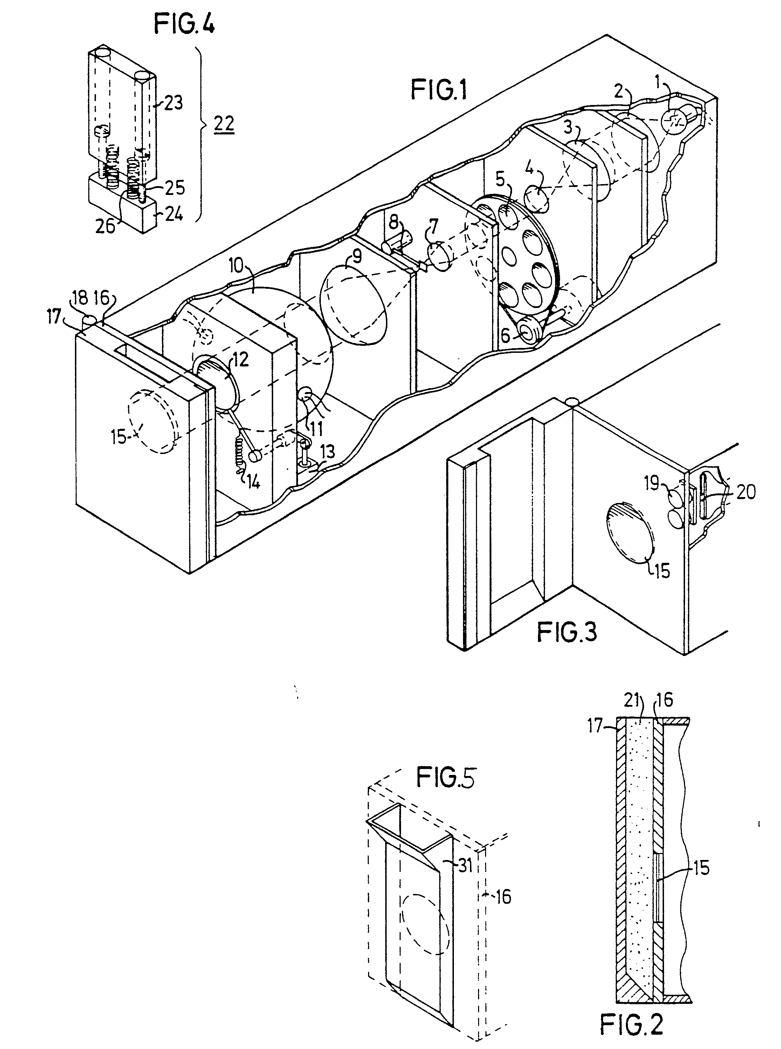

Figur 1 stellt einen erfindungsgemässen IR-Analysator dar. Eine Lichtquelle 1, beispielsweise eine gewöhnliche Glühlampe, Halogen-, Xenon- oder Leuchtstofflampe, sendet Licht durch eine aus zwei bikonvexen Linsen 2 und 3 zusammengesetzte Optik, eine Kondensorlinse 4 und eine Lichtfilterscheibe 5. Diese Scheibe enthält mehrere optische monochromatische Filter, die nur für je eine IR-Wellenlänge durchlässig sind. Diese Scheibe 5 wird von einem elektrischen Schrittmotor bei 6 stufenweise gedreht, wobei sie in 5 bis 15 Sekunden eine volle Umdrehung durchführt, welche sich in diesem Falle aus acht Drehschritten zusammensetzt. Bei jeder Pause zwischen zwei Drehschritten befindet sich nur ein einziges der acht Filter der Filterscheibe im Strahlengang. Hinter der Scheibe wird das monochromatische Licht von einer Linse oder einer zusammengesetzten Optik 7 fokusiert. Im Brennpunkt befindet sich ein Lichtzerhacker in Form einer piezoelektrisch oder magnetisch bewegten Fahne 8, welche das Licht periodisch mit einer höheren Frequenz als 100 Hz, zweckmässigerweise 200 Hz, abschirmt, so dass vom Licht erzeugte elektrische Signale diese Frequenz erhalten, um leichter verstärkt werden zu können.

2

Dieses intermittierende Licht setzt seinen Weg durch einen kollimierenden Kondensor 9 in eine Kammer 10 hinein fort, die hier eine hohle sog. Ulbrichtsche Kugel 10 mit einer dem Kondensor 9 zugekehrten Eintrittsöffnung für das einfallende Licht ist. Der Kondensor 9 gibt den Lichtstrahl mit grösserem Querschnitt als das Filter ab. Gegenüber der Eintrittsöffnung ist die Kugel 10 mit einem entsprechenden, mindestens ebenso grossen Fenster 15 versehen. Dieses Fenster ist durch eine in ihrer eigenen Ebene schwenkbare lichtundurchlässige Scheibe 12 optisch abdeckbar, die mittels einem Elektromagneten 13 oder einer mechanischen oder elektrischen Kupplung mit der Filterscheibe 5 synchron hiermit zwischen einer völlig lichtabschirmenden und einer das Fenster völlig freigebenden Stellung schwenkbar ist.

Hier wird nur schematisch ein Elektromagnet 13 zum Wegschwenken der Scheibe 12 von der Öffnung, d. h. zur Freigabe des Lichts, gezeigt, während bei stromlosem Magneten das Zurückschwenken der Scheibe durch eine Rückholfeder 14 erfolgt. Die der genannten Eintrittsöffnung und dem Kondensor 9 zugekehrte Seite der Scheibe kann eben sein oder mit ungefähr gleichem Halbmesser wie die Kugelinnenseite gewölbt sein und dient als die weiter unten erläuterte Referenz. Diese Oberfläche der Scheibe und die Innenseite der Kugel sind vorzugsweise matt vergoldet, um diffus reflektierend, chemisch resistent und nichtalternd zu sein. Diese als Referenz dienende Oberfläche der Scheibe 12 kann jedoch aus einem anderen beständigen Werkstoff als die Innenseite der Kugel bestehen, z. B. aus Polytetrafluoräthylen.

Bei weggeschwenkter Scheibe 12 fällt das Licht durch das Glasfenster 15 auf eine Probe 21 (Fig. 2) in einem Halter oder Behälter für die Probe, wobei das Fenster als Bestandteil des Probenbehälters betrachtet werden kann. Zu diesem Behälter gehört auch ein mit dem Fenster und dem Analysatorkörper fest verbundener, aber ggf. abnehmbarer, z. B. abschraubbarer Teil 16 und eine bewegliche Wand 17, die leicht anbringbar und vom Teil 16 leicht abnehmbar, schwenkbar oder in einer Führung verschiebbar ist, möglichst ohne Verwendung irgendeiner Art von Werkzeug. Zu diesem Zweck können die beiden Glieder 16 und 17 mit einem Scharnier 18 nach Fig. 1 und 3 miteinander verbunden sein oder mittels Raste, Klemmvorrichtung, Magnet, Haken oder anderem geeigneten bekannten Mittel. Die der Probe zugekehrte Oberfläche des Fensters 15 sollte möglichst mit der das Fenster umgebende Wand des Teiles 16 fluchten, so dass diese Innenwand des Probenbehälters keine Absätze aufweist und durch Abwischen leicht zu reinigen ist.

Figur 2 ist ein senkrechter Schnitt durch den Probenbehälter in der Ebene des Strahlengangs.

Figur 3 zeigt ein Ausführungsbeispiel des Probenbehälters mit dem Scharnier 18 zwischen dem festen Teil 16 und der beweglichen Wand 17, die in Fig. 1 in geschlossener und in Fig. 3 in offener Stellung dargestellt ist. Die bewegliche Wand ist ferromagnetisch oder enthält ein ferromagnetisches Element. In zum Teil 16 hin geschwenkter Stellung, in welcher der Teil 16 und die Wand 17 gegeneinander anliegen, wird die Wand 17 durch einen Dauermagneten 19 am Teil 16 festgehalten. Diese Einrichtung kann natürlich auch umgekehrt sein, d. h. so dass der Teil 16 teilweise oder ganz ferromagnetisch ist und mit einem Dauermagneten in der Wand 17 zusammenwirkt. Ferner ist ein Zungenrelaiskontakt 20 vorgesehen, der den Strom der Lichtquelle unterbricht und/oder die Erregung des die Referenzscheibe 12 betätigenden Elektromagneten so steuert, dass dieser Magnet unwirksam wird, wenn der Probenbehälter geöffnet wird, indem seine Wand 17 weggeschwenkt wird. Hierdurch wird verhindert, dass Fremdlicht in die Kugel 10 eindringt und die darin befindlichen Fotozellen 11 trifft, welche durch Tageslicht und anderes Licht, welches UV enthält, verschlechtert werden können. Der Kontakt 20 kann ferner so geschaltet sein, dass er bei offenem Probenbehälter auch den Stromkreis des Schrittmotors 6 unterbricht. Das Unterbrechen des Stromkreises der Lichtquelle hat u. a. den Zweck, die Fotozellen zu verdunkeln und unnötige Wärmeerzeugung zu vermeiden.

Hier sei erwähnt, dass die magnetische Steuerung des Kontakts 20 in verschiedener Weise erfolgen kann. Beispielsweise kann der magnetische Kreis des Dauermagneten 19 beim Schliessen des Probenbehälters ganz oder teilweise geschlossen werden, so dass das den Zungenrelaiskontakt 20 steuernde Magnetfeld geschwächt wird. Ist der Dauermagnet 19 dagegen in der beweglichen Wand 17 angeordnet, kann der Zungenrelaiskontakt durch Erhöhung des ihn steuernden Magnetfeldes betätigt werden, wenn die Wand 17 gegen den Teil 16 angelegt wird.

Wenn die Wand 17 mit ihren Randzonen gegen den Teil 16 anliegt, sollten dieser Teil und die Wand « mehldicht » gegeneinander anliegen, ausgenommen die Einfüllöffnung an der einen, in der Zeichnung oberen Schmalseite eines Zwischenraums, der einen rechteckigen Schacht bildet, aber nach unten hin sich in der einen oder in beiden senkrechten Schachtebenen etwas verjüngen kann. Die Innenkanten des Schachts sind vorzugsweise etwas gerundet, um eine Reinigung des Schachts zu erleichtern. Das Mehl, d. h. die Probe, wird durch die Einfüllöffnung oben am Schacht in den Behälter gefüllt, vorzugsweise durch einen an die längliche Form der Einfüllöffnung angepassten Trichter. Der Schacht sollte keine solchen Vorsprünge oder Vertiefungen enthalten, die eine gleichmässige Verteilung des Mehls im Schacht oder dessen Reinigung nach Verwendung erschweren könnten.

Das in den Schacht gefüllte Mehl wird mit gewissem günstigen Druck mittels einem Verdichter zusammengedrückt, z. B. mit einem Verdichter 22 nach Fig. 4, der im wesentlichen aus zwei verschieden grossen rechteckigen Körpern 23 und 24 und aus dieselben beweglich miteinander verbindenden Führungsstiften 25 und Druckfedern 26 besteht. Die beiden Körper 23 und 24 können die gleiche Querschnittform haben wie die Einfüllöffnung des Behälters 15-17 und dessen Schacht. Bei gefülltem Probenbehälter steckt man den Verdichter 22 mit der unteren Seite des Körpers 24 in die Öffnung und

3

drückt bis die beiden Körper 23 und 24 (oder Anschläge auf ihnen) kaum oder schwach gegeneinander anliegen. Das Mehl im Behälter wird dadurch einem Druck ausgesetzt, der durch die Federn 26 in zusammengedrückter Stellung der beiden Teile bestimmt wird. Dieser Druck und die Oberflächenbeschaffenheit sind für eine zuverlässige Messung ziemlich wichtig, obwohl der Druck nicht kritisch ist, da man sonst verschiedene Messwerte für verschiedene Proben von ein und derselben Mehlmenge erhalten kann, d. h. die Messgenauigkeit des Analysators wäre nicht besser als der grösste Unterschied zwischen den von mehreren Proben ein und derselben Mehlware erhaltenen Werten.

Der Boden des im übrigen ungefähr rechteckigen Schachtes kann nach unten gegen den Teil 16 hin geneigt sein, z. B. um etwa 30-60°. Im Prinzip ist es unwichtig, ob nur der Teil 16 oder nur die Wand 17 oder beide einen den Schacht (Zwischenraum) bildenden Hohlraum nach Fig. 1-3 bilden. Unabhängig davon, ob der Schachtboden geneigt ist oder nicht, kann er durch eine federnde Klappe gebildet werden, die nachgibt und einen Teil der eingefüllten Mehlprobe nach aussen hindurchlässt, falls die Probe (21 in Fig. 2) zu stark zusammengedrückt wird. Dies könnte eintreten, wenn man von Hand den Verdichter 22 nach Fig. 4 benutzt und ihn in den Schacht weiter hineindrückt, nachdem die beiden Körper 23 und 24 des Verdichters bereits gegeneinander anliegen. An sich braucht aber der Verdichter selbst nicht federnd zusammendrückbar sein, sondern kann aus einem starren Körper bestehen, so dass die Probe mit einer Kraft zusammengedrückt wird, die durch die Federkraft des federnden Schachtbodens bestimmt wird. Die Federkraft kann in bekannter Weise durch Schwerkraft ersetzt oder ergänzt werden, z. B. durch ein Gegengewicht.

Verwendung und Arbeitsweise des Analysators sind wie folgt. Der Schacht (Zwischenraum) des Probenbehälters zwischen den Gliedern 17 und 15-16 wird mit Mehl gefüllt. Die aus diesem Mehl bestehende Probe wird mit dem Verdichter 22 nach Fig. 4 zusammengedrückt, so dass sie mit gewissem Druck dicht direkt gegen das Fenster 15 anliegt, welches das einzige Glas im Strahlengang zwischen der Probe und den Fotozellen 11 ist. Mit einer Schalttaste wird die Lichtquelle 1 eingeschaltet und der Schrittmotor 6 dreht die Filterscheibe in eine Stellung, in der eines ihrer Filter im Strahlengang liegt. Ferner schwenkt der Elektromagnet 13 die Referenzscheibe 12 vom Fenster 15 der Kugel 10 seitlich weg, so dass das vom Filter und Kondensor kommende monochromatische kollimierte Licht die Probe trifft. Das von der Probe reflektierte Licht wird in der Kugel durch die mattvergoldete Innenwand der Kugel mit diffuser Streuung zu den Fotozellen 11 reflektiert. Diese geben daher ein entsprechendes Wechselstromsignal mit einer Frequenz von — in diesem Beispiel— 200 Hz infolge der Vibrationen des Lichtzerhackers 8 ab. Das elektrische Fotozellensignal wird verstärkt, gefiltert, gleichgerichtet und in ein digitales Signal umgewandelt, das einem Mikroprozessor zugeführt und dort gespeichert und ausgewertet wird.

Während das genannte Filter der Filterscheibe 5 immer noch in den Strahlengang eingeschaltet ist, wird der Elektromagnet 13 abgeschaltet, so dass die Feder 14 die Referenzscheibe 12 in die Stellung zurückholt, in der die Scheibe die Probe gegen das Licht abschirmt und mit ihrer mattvergoldeten Oberfläche Referenzlicht in die Kugel hinein reflektiert, wo dieses Licht diffus die Fotozellen 11 erregt. Diese geben jetzt daher ein elektrisches Referenzsignal ab, das ebenfalls ein Wechselstromsignal ist und im Prinzip in gleicher Weise wie das Probensignal in oben genannter Art weiterverarbeitet wird. Erwünschtenfalls kann man den Analysator so vorsehen oder steuern, dass man mehrere Probensignale abwechselnd mit Referenzsignalen jedesmal mit Licht von ein und demselben Filter bei stillstehender Filterscheibe 5 erhält, ehe diese Scheibe vom Schrittmotor 6 um einen Schritt weitergedreht wird und das nächste Filter in den Strahlengang einschaltet.

Zur Untersuchung ein und derselben Probe wird obiges für jedes Filter der Reihe nach wiederholt. Fig. 1 zeigt die Filterscheibe 5 mit acht Filtern, aber je nach Art der Probe und gewünschter Analyse braucht die gesamte Messung natürlich nicht unbedingt mit sämtlichen Filtern erfolgen.

Nachstehend wird angenommen, dass Proben- und Referenzmessung je zweimal mit jedem von sechs Filtern erfolgt. Die jeweils erste und zweite Messung wird mit Index 1 bzw. 2 gefolgt von der Nummer des Filters (Nr. 1 bis 6 für sechs Filter) bezeichnet. Das Probensignal wird mit P und das Referenzsignal mit R bezeichnet.

Ein an den eigentlichen Analysator 1-18 angeschlossener Mikroprozessor berechnet den Wert

$$k_0 + k_1 \log \frac{R_{11} + R_{21}}{P_{11} + P_{21}} + k_2 \log \frac{R_{12} + R_{22}}{P_{12} + P_{22}} \cdots\cdots k_6 \log \frac{R_{16} + R_{26}}{P_{16} + P_{26}}$$

wobei $k_0 \ldots k_6$ Konstanten sind, die nach dem Verfahren des kleinsten Quadrats bestimmt werden. Z. B. die Bezeichnung $R_{12}$ bedeutet daher die erste Messung (Nr. 1) mit dem zweiten Filter (Nr. 2). Der Wert des obigen mathematischen Ausdrucks wird also dadurch erhalten, dass in erster Linie der Logarithmus des Quotienten : Summe der beiden Referenzsignalamplituden dividiert mit der Summe der zwei Probensignalamplituden, gebildet wird. Eine Quotientenberechnung im engeren Sinne des Wortes ist jedoch nicht unbedingt nötig, da der Logarithmus eines Quotienten bekanntlich gleich ist dem Logarithmus des Zählers minus dem Logarithmus des Nenners.

Der nach obiger Formel erhaltene Wert gibt im hier beschriebenen Beispiel den relativen Eiweissgehalt (Proteingehalt) von Mehl an, wenn die Probe aus Getreidemehl besteht und mit sechs verschiedenen IR-Wellenlängen der Reihe nach bestrahlt wird. Diese sechs Lichtwellenlängen werden durch die sechs benutzten Filter bestimmt und sind jede für sich so gewählt, dass sie sich für die gewünschte Proteinanalyse eignen.

Mit einem erfindungsgemässen Analysator kann man eine sehr hohe Genauigkeit erzielen. Nichtkompensierte Temperaturdrift verschlechtert bekanntlich die Genauigkeit von Messgeräten wie Analysatoren obiger Art. Bei einem erfindungsgemässen Analysator wird die Temperaturdrift vorzugsweise, jedoch nicht unbedingt, dadurch ausgeglichen, dass Schwankungen des von der Referenz 12 erzeugten Referenzsignals R vom Datenverarbeiter in solcher Weise behandelt werden, dass eine entsprechende kompensierende Änderung des Probensignals dadurch hervorgerufen wird, dass der Verstärkungsfaktor in Abhängigkeit von etwaigen Änderungen des Referenzsignals geregelt wird. Die Einrichtung kann erforderlichenfalls auch gegen etwaige Lichtstärkeschwankungen der Lichtquelle 1 unempfindlich gemacht werden. Wird die Temperaturdrift in soeben genannter Art kompensiert, kann man leicht vermeiden, dass der Analysator den Einfluss von Lichtstärke- und Temperaturänderungen auf das elektrische Referenzsignal sozusagen miteinander verwechselt.

Fig. 5 zeigt, wie ein erfindungsgemässer Analysator zur Prüfung oder Analyse von Mehl o. ä. in einem im Querschnitt rundherum geschlossenen Mehlförderkanal 27 benutzt werden kann, d. h. in einem Kanal 27, der meistens aus einer runden oder rechteckigen Rohrleitung, z. B. aus einem Fallschacht, besteht. Diese Rohrleitung ist an einer Stelle so zweigeteilt, dass ein Zweigkanal 28 gebildet wird, durch den ein Teil des geförderten Mehls entweder ständig oder vorzugsweise nur zwischen zwei IR-Messungen fliesst. Die Zweigleitung 28 enthält eine vorzugsweise durch ein elektrisches Steuerglied 30 verstellbare Absperrklappe 29 oder ein ähnliches Absperrorgan, z. B. Absperrschieber. Das Fenster 15 nach Fig. 1-3 ist in den Zweigkanal 28 so eingebaut, dass die Innenwand des Kanals und die dem Kanal zugekehrte Oberfläche des Fensters in der gleichen Ebene liegen, also miteinander fluchten. Der Probenbehälter wird hier durch den mit dem Fenster 15 versehenen Teil des Kanals und durch die Klappe 29 gebildet, welche in geschlossenem Zustand den Boden des Behälters bildet. Die Einfüllöffnung des Behälters besteht aus dem inneren Hohlraum des rohrförmigen Zweigkanals 28 oberhalb des Fensters. Das Zusammendrücken der Mehlprobe vor und während der Analyse kann durch das Eigengewicht der im Zweigkanal oberhalb der Klappe und des Fensters befindlichen Mehlmenge bewirkt werden oder durch eine besondere Verdichtungseinrichtung, die nicht näher beschrieben zu werden braucht. Ebenzo wie im weiter oben beschriebenen Falle kann die Klappe 29 eine federnde Klappe sein, die sich selbsttätig öffnet, wenn der Druck der Mehlprobe den gewünschten oder erforderlichen Wert überschreitet.

Gegebenenfalls kann man aber je nach Art des Förderguts und/oder sonstiger Verhältnisse auf den Zweigkanal 28 verzichten und eine Zone des Förderkanals 27 selbst als Probenbehälter benutzen, wenn der Analysator 1-16 mit seinem Fenster 15 an dieser Zone direkt angebracht wird und die Absperrklappe 29, sofern sie nicht ganz entbehrlich oder durch eine Kanalverengung ersetzbar ist, im Kanal 27 in gleicher Weise unterhalb des Fensters 15 vorgesehen ist wie in Fig. 5 für den Zweigkanal 28 dargestellt. Bildet der Förderkanal 27 einen Fallschacht, so dass das Fördergut (Mehl) durch sein Eigengewicht hindurch gleitet, kann der Kanal 27 oberhalb des Fensters mit einer Krümmung solcher Art versehen werden, dass im wesentlichen fast nur die Mehlmenge zwischen der Kanalkrümmung und dem Fenster auf die Mehlprobe 21 vor dem Fenster zusammendrückend wirkt. Wenn der Kanal 27 oberhalb der Klappe genügend kurz ist oder genügend geneigt, also nicht senkrecht ist, kann die genannte Krümmung ggf. überflüssig sein. Erfolgt der Mehlvorschub im Kanal 27 zwangsweise mit zu hohem Druck gegen die geschlossene Absperrklappe 29, kann man, wenn der Förderdruck statisch ist, also bei geschlossener Klappe nicht ständig zunimmt, den Kanal mit einer Querschnittserweiterung an und über der Absperrklappe versehen. Wird der Druck dynamisch erzeugt, so dass er bei zunehmendem Widerstand im Kanal zunimmt, kann man einen offenen Kanal in oder neben der Klappe vorsehen oder die Klappe wie ein Ueberdruckventil federnd ausführen, um einen Ueberdruck auf die Mehlprobe zu verhindern. Es bestehen aber noch viele andere Möglichkeiten.

Fig. 6 zeigt einen Probenbehälter für einzelne Mehlproben, so wie es in Fig. 1-3 der Fall ist. Der Behälter ist aber besonders mit seiner oben trichterförmig geformten äusseren Wand 31, die der Wand 17 in Fig. 1-3 entspricht, verschiebbar wie ein Gleitschieber ausgeführt, ungefähr wie die Kassette für fotografische Glasplatten in einer Plattenkamera oder Projektor.

Ein erfindungsgemässer Analysator in der mit Varianten oben beschriebenen Bauart kann billiger, mit höherer Messgenauigkeit und vielseitigeren Verwendungsmöglichkeiten hinsichtlich der Konsistenz von zu untersuchenden Proben ausgeführt werden als die handelsüblichen IR-Analysatoren ähnlicher Bauart zur Untersuchung des relativen Protein- und/oder Wassergehalts von Getreidemehl und anderen Lebensmitteln mit nicht fester Konsistenz.

**Patentansprüche**

1. Infrarotanalysator zur relativen Mengenbestimmung eines oder mehrerer bestimmter Stoffe in einer Probe (21), besonders in Lebensmitteln wie Mehl, mit einer Infrarot-Lichtquelle (1), die abwechselnd die Probe (21) und eine Referenz (12) bestrahlt, mit einer Fotozelleneinrichtung (11), die von der Probe und der Referenz diffus reflektiertes Licht empfängt und in entsprechende elektrische Signale umwandelt, und mit einem mit einem lichtdurchlässigen Fenster (15) versehenen Behälter (16, 17) für die Probe (21), welcher Behälter aus dem genannten Fenster (15) und einem das Fenster umgebenden Wandbereich (16) sowie aus einer dem Fenster entgegenstehenden Wand (17) besteht, wobei das Fenster und die Wand (17) auseinanderbewegt und zusammengesetzt werden können und so geformt sind, dass ein zur Aufnahme der Probe (21) geeigneter Zwischenraum zwischen dem Fenster (15) und der genannten Wand (17) vorhanden ist, dadurch gekennzeichnet, dass dieser Zwischenraum eine zum Einfüllen der Probe dienende, gegebenenfalls verschliessbare Öffnung in solcher Lage aufweist, dass die Probe nur in einer mit der Fensterebene ungefähr parallelen Richtung in den Zwischenraum einfüllbar ist, dass ein Probenverdichter (22) vorgesehen ist, der, falls erforderlich, die Probe durch die Öffnung hindurch zusammendrückt, dass die Wand (17) beweglich wie eine Klappe mit dem Wandbereich (16) schwenkbar verbunden ist, und dass das Fenster (15) beim Bestrahlen der Probe (21) der einzige Gegenstand ist, der von der die Probe bestrahlenden Lichtquelle nach Kollimierung des Lichts getroffen wird.

2. Analysator nach Anspruch 1, dadurch gekennzeichnet, dass die genannte Einfüllöffnung für die Probe (21) die Form eines Schlitzes mit im wesentlichen gleicher Fläche und Form seines Querschnitts wie der Querschnitt des genannten Zwischenraumes hat.

3. Analysator nach einem der vorangehenden Ansprüche, dadurch gekennzeichnet, dass der genannte Zwischenraum an seiner der Einfüllöffnung entgegengesetzten Seite mit einem federnden Deckel oder Klappe verschlossen ist, so dass die durch die Öffnung eingefüllte Probe (21) bei zu hartem Druck auf dieselbe diesen Deckel oder Klappe öffnet und teilweise austritt.

4. Analysator nach einem der vorangehenden Ansprüche, dadurch gekennzeichnet, dass der Probenverdichter (22) einen Querschnitt von im wesentlichen gleicher Fläche und Form wie die Öffnung und der Zwischenraum besitzt, und aus zwei miteinander beweglich verbundenen Teilen (23, 24) besteht, die in der Verdichtungsrichtung federnd (26) gegeneinander verschiebbar sind und daher beim Einschieben des Verdichters in den die Probe enthaltenen Zwischenraum die Probe durch Federkraft zusammendrücken.

5. Analysator nach einem der vorangehenden Ansprüche, dadurch gekennzeichnet, dass die Referenz aus einem Element (12) mit einer vom Fenster (15) abgewandten Oberfläche mit den gewünschten referenzbildenden Eigenschaften gebildet ist, dass dieses Element im Strahlengang am Fenster zwischen einer das Fenster abschirmenden und einer das Fenster freigebenden Stellung beweglich ist, und dass der Strahlengang zwischen dem Fenster und dem Element gerade ist und kein festes oder flüssiges Material enthält.

### Claims

1. Infrared analyzer for determining the relative amounts of one or more specific substances in a sample (21), especially in edibles such as flour, with an infrared light source (1), which alternatingly illuminates the sample (21) and a reference (12), with a photocell arrangement (11) which receives diffuse reflected light from the sample and the reference converting it into a corresponding electrical signal, and with a container (16, 17) provided with a transparent window (15), for the sample (21), said container consisting of said window (15) and a wall area (16) surrounding said window as well as a wall (17) opposite said window, said window and said wall (17) being able to be moved apart and together and being so formed that an intermediate space is provided between the window (15) and the said wall (17) for receiving the sample (21), characterized in that this space has in such a position a possibly closable opening for filling with the sample, that the sample can only be filled into the space in a direction approximately parallel to the window surface, that a sample compacter (22) is provided which, if necessary, compresses the sample through the opening, that the wall (17) is hingedly connected to the wall area (16), and that the window (15), when the sample (21) is illuminated, is the only object struck by the sample illuminating light source after collimation.

2. Analyzer according to Claim 1, characterized in that said filler opening for the sample (21) has the form of a slot with substantially the same cross sectional area and shape as the cross section of said intermediate space.

3. Analyzer according to any one of the preceding claims, characterized in that said space is closed at its side opposite the filling opening by a spring cover or hatch, so that if a sample (21) is filled through the opening at excessive pressure this cover or hatch will open and the sample will partially flow out.

4. Analyzer according to any one of the preceding claims, characterized in that the sample compacter (22) has a cross section of essentially the same area and shape as the opening and the intermediate space, and consists of two parts (23, 24) movably joined to each other, and which in the compacting direction are resiliently (26) displaceable towards each other and hence upon insertion of the compacter into the space containing the sample, compresses the sample by spring force.

5. Analyzer according to any one of the preceding claims, characterized in that the reference is formed of an element (12) having a surface facing away from the window (15) and having the desired reference-forming properties, that this element, in the light path to the window can be moved between a position blocking the window and a position not blocking the window, and that the light path between the window and the element is straight and contains no solid or liquid materials.

**Revendications**

1. Analyseur infrarouge pour la détermination quantitative relative d'une ou de plusieurs substances déterminées dans une éprouvette (21), en particulier dans des produits alimentaires tels que la farine, comportant une source (1) de lumière infrarouge qui éclaire alternativement l'éprouvette (21) et une référence (12), un dispositif à cellules photo-électriques (11) qui reçoit de la lumière à réflexion diffuse provenant de l'éprouvette et de la référence, et la convertit en des signaux électriques correspondants, ainsi qu'un réceptacle (16, 17) de l'éprouvette (21) muni d'une fenêtre translucide (15), ce réceptacle comprenant la fenêtre précitée (15) et une zone de paroi (16) qui entoure cette fenêtre, ainsi qu'une paroi (17) opposée à ladite fenêtre, la fenêtre et la paroi (17) pouvant être dissociées et combinées, et étant configurées de telle sorte qu'un espace intermédiaire, approprié pour recevoir l'éprouvette (21), soit présent entre la fenêtre (15) et la paroi précitée (17), caractérisé par le fait que cet espace intermédiaire présente un orifice éventuellement obturable, servant à déverser l'éprouvette et occupant une position telle que cette éprouvette puisse être déversée dans l'espace intermédiaire seulement dans une direction approximativement parallèle au plan de la fenêtre ; par le fait qu'il est prévu un compacteur (22) d'éprouvette qui, si nécessaire, comprime l'éprouvette à travers l'orifice ; par le fait que la paroi (17) est reliée à la zone de paroi (16) avec faculté de pivotement, en étant mobile à la manière d'un volet ; et par le fait que, lors de l'éclairage de l'éprouvette (21), la fenêtre (15) est le seul objet à être atteint par la source lumineuse qui éclaire l'éprouvette, après collimation de la lumière.

2. Analyseur selon la revendication 1, caractérisé par le fait que l'orifice susmentionné de déversement de l'éprouvette (21) revêt la forme d'une fente, dont la section présente une superficie et une forme sensiblement identiques à celles de la section de l'espace intermédiaire précité.

3. Analyseur selon l'une des revendications précédentes, caractérisé par le fait que l'espace intermédiaire susmentionné est obturé par un couvercle ou un volet élastique sur son côté opposé à l'orifice de déversement, si bien que l'éprouvette (21) déversée par l'orifice ouvre ce couvercle ou volet lorsqu'elle est soumise à une trop forte pression, et s'échappe en partie.

4. Analyseur selon l'une des revendications précédentes, caractérisé par le fait que le compacteur (22) d'éprouvette possède une section ayant une superficie et une forme sensiblement identiques à celles de l'orifice et de l'espace intermédiaire, et se compose de deux parties (23, 24) reliées l'une à l'autre avec faculté de mouvement, qui peuvent coulisser élastiquement (26) l'une vers l'autre dans la direction du compactage et qui, de ce fait, lorsque le compacteur est introduit dans l'espace intermédiaire renfermant l'éprouvette, compriment cette éprouvette par une force élastique.

5. Analyseur selon l'une des revendications précédentes, caractérisé par le fait que la référence est formée par un élément (12) doté d'une surface qui est tournée à l'opposé de la fenêtre (15), et présente les propriétés souhaitées de formation d'une référence ; par le fait que cet élément est mobile sur la fenêtre dans la trajectoire des rayonnements, entre une position obturant ladite fenêtre et une position dégageant cette fenêtre ; et par le fait que la trajectoire des rayonnements, entre la fenêtre et l'élément, est rectiligne et ne renferme aucun matériau solide ni liquide.

FIG.4

FIG.1

FIG.3

FIG.5

FIG.2